# EUROPÄISCHE PATENTSCHRIFT

(11) **EP 2 565 669 B1**
(45) Veröffentlichungstag und Bekanntmachung des Hinweises auf die Patenterteilung: **25.03.2020**
(21) Anmeldenummer: 12181956.9
(22) Anmeldetag: 28.08.2012
(51) Int. Cl.: G01S 7/497, G01S 7/481, G01S 7/487, G01S 17/10

(54) **Verfahren zur Unterdrückung eines Echosignals**
Method for suppressing an echo signal
Procédé destiné à supprimer un échosignal

(30) Priorität: 29.08.2011 AT 12322011
(43) Veröffentlichungstag der Anmeldung: 06.03.2013
(73) Patentinhaber: Swarovski-Optik KG., 6067 Absam (AT)
(72) Erfinder: Lancaster, Gavin, 4792 Münzkirchen (AT); Roider, Konrad, 6068 Mils (AT); Sewald, Dieter, 9492 Eschen (LI)
(74) Vertreter: Burger, Hannes

(56) Entgegenhaltungen:
- EP-A1- 1 288 676
- EP-A2- 1 901 092
- WO-A1-2005/054902
- DE-A1- 10 153 742
- DE-A1-102008 018 718
- US-A1- 2003 035 097
- US-A1- 2008 143 998
- US-B1- 6 650 404

## Beschreibung

Die Erfindung betrifft ein Verfahren zum Unterdrücken eines optischen Übersprechers in einer Entfernungsmesseinrichtung in einer Entfernungsmesseinrichtung gemäß dem Oberbegriff von Anspruch 1.

Ferner betrifft die Erfindung eine Entfernungsmesseinrichtung mit zumindest einem lichtempfindlichen Empfänger und zumindest einer Laserlichtquelle als Sender, gemäß dem Oberbegriff von Anspruch 3.

Darüber hinaus betrifft die Erfindung eine Fernoptische Vorrichtung, insbesondere Fernrohr oder Zielfernrohr.

Bei gattungsgemäßen Entfernungsmesseinrichtungen, welche auch als Laser Rangefinder bezeichnet werden, wird eine Entfernungsmessung durchgeführt, indem ein Laserpuls bzw. Pulszüge von einem Sender ausgestrahlt werden, vom Zielobjekt reflektiert werden, von einem Empfänger detektiert werden, und anhand der zeitlichen Verzögerung zwischen den Sende- und Empfangspulsen die Laufzeit und daraus der zurückgelegte Weg bestimmt wird. Ein Verfahren und eine Entfernungsmesseinrichtung der eingangsgenannten Art sind aus der US2008/143998 A1 bekannt geworden

Bei einer gattungsgemäßen Entfernungsmesseinrichtung können Sender und Empfänger zumindest teilweise in demselben optischen Pfad angeordnet sein. Sind nun Sender und Empfänger in einem Gehäuse und vielleicht sogar an der selben (Prismen)Optik, wie beispielsweise aus der WO 2009094687 A2 bekannt, angebracht, so kann es passieren, dass es zu direkten optischen Übersprechern kommt. Das bedeutet, dass ein kleiner Teil der vom Sender abgestrahlten Laserpulse durch interne Streuung, Reflexion, etc. den empfindlichen Empfänger direkt erreichen und ein Messsignal erzeugt. Dieses falsche Messsignal ist üblicherweise größer als die Signale von einem entfernteren Zielobjekt und stören/verfälschen deshalb die Messung. Da dieses Streulicht zeitlich immer als erstes vorhanden ist, sind davon nur Nahbereichsmessungen (bis einige 100m) betroffen. Unter dem Begriff Störechosignal werden in diesem Dokument alle durch interne Reflexion, Streuung oder andere Störeinflüsse zu dem Empfänger gelangenden Anteile des von dem Sender emittierten Lichts bezeichnet.
Das optische Übersprechen kann durch bauliche Maßnahmen (zB. Blenden, Geometrie) beeinflusst werden. Elektronisch kann zB. ein erstes zeitliches Fenster definiert werden, in dem zB. der Empfänger oder der Verstärker nicht aktiv sind, da hier der Übersprecher vorhanden ist. Erst nach Ablauf dieses Fensters wird dann eine Entfernungsmessung durchgeführt. Dies hat aber den Nachteil, dass in diesem Fenster überhaupt keine Messung durchgeführt werden kann. In der US 7,599,045 B2 und der US2008/143998 A1 wird dieses ,blinde' Fenster realisiert, in dem der in diesem zeitlichen Fenster liegende und abgezweigte Teil vom detektierten Signal invertiert und wieder zum Messsignal addiert wird, so dass das Summensignal zu Null wird. Nachteilig an der bekannten Ausführungsform ist es, dass bedingt durch das 'blinde' Fenster nur Entfernungen von deutlich mehr als 100 m gemessen werden können.

Aus der WO2005/054902 A1 ist ein Entfernungsmesser bekannt geworden, bei welchen ein Signal erfasst wird, welches der Intensität eines von einem entfernten Objekt reflektierten Lichtbündels entspricht. Die US6650404 B1 hat einen Laser-Rangefinder zum Gegenstand, bei welchem ein von einem entfernten Objekt reflektiertes Signal zur Verbesserung eines Signal/Rauschverhältnisses einer Hochpassfilterung unterworfen wird.
Der Erfindung liegt daher die Aufgabe zugrunde, den Einfluss des optische Übersprechens durch elektronische Maßnahmen so weit zu minimieren, dass auch bei kurzen Messdistanzen (<100m) eine Entfernungsmessung möglich ist, ohne dass Nutzechos unzulässig unterdrückt werden.

Diese Aufgabe wird mit einem Verfahren der eingangs genannten Art erfindungsgemäß durch die Merkmale des kennzeichnenden teils von Anspruch 1 gelöst.

Gemäß der bevorzugten Ausführungsform der Erfindung werden das von dem Empfänger erzeugte Signal und das Gegensignal voneinander abgezogen. Auf diese Weise lassen sich Störeinflüsse auf einfache Weise verringern bzw. eliminieren.

Die eingangs genannte Aufgabe lässt sich auch mit einer Entfernungsmesseinrichtung der eingangs genannten Art erfindungsgemäß durch die Merkmale des kennzeichnenden Teils von Anspruch 3 gelöst.

Gemäß einer vorteilhaften Weiterbildung der Erfindung kann die Entfernungsmesseinrichtung dazu eingerichtet sein, das von dem Empfänger erzeugte Signal und das Gegensignal voneinander abzuziehen.

Die oben genannte Aufgabe lässt sich auch mit einer fernoptischen Vorrichtung der eingangs genannten Art lösen, welche eine erfindungsgemäße Entfemungsmesseinrichtung aufweist, wobei Sender und Empfänger zumindest teilweise in demselben optischen Pfad angeordnet sind.

Die Erfindung samt weiteren Vorteilen wird im Folgenden anhand einiger nicht einschränkender Ausführungsbeispiele näher erläutert, welche in den Zeichnungen dargestellt sind.

Es zeigen jeweils in stark schematisch vereinfachter Darstellung:
- Fig. 1: ein Blockschaltbild einer erfindungsgemäßen Entfernungsmesseinrichtung;
- Fig. 2: einen Signalverlauf eines aus einer Überlagerung zwischen einem Echosignal und einem Gegensignal resultierenden Signals;
- Fig. 3: ein Blockdiagramm einer weiteren Ausführungsform der Erfindung.

Einführend sei festgehalten, dass in den unterschiedlich beschriebenen Ausführungsformen gleiche Teile mit gleichen Bezugszeichen bzw. gleichen Bauteilbezeichnungen versehen werden, wobei die in der gesamten Beschreibung enthaltenen Offenbarungen sinngemäß auf gleiche Teile mit gleichen Bezugszeichen bzw. gleichen Bauteilbezeichnungen übertragen werden können. Auch sind die in der Beschreibung gewählten Lageangaben, wie z.B. oben, unten, seitlich usw. auf die unmittelbar beschriebene sowie dargestellte Figur bezogen und sind bei einer Lageänderung sinngemäß auf die neue Lage zu übertragen. Weiters können auch Einzelmerkmale oder Merkmalskombinationen aus den gezeigten und beschriebenen unterschiedlichen Ausführungsbeispielen für sich eigenständige, erfinderische oder erfindungsgemäße Lösungen darstellen.

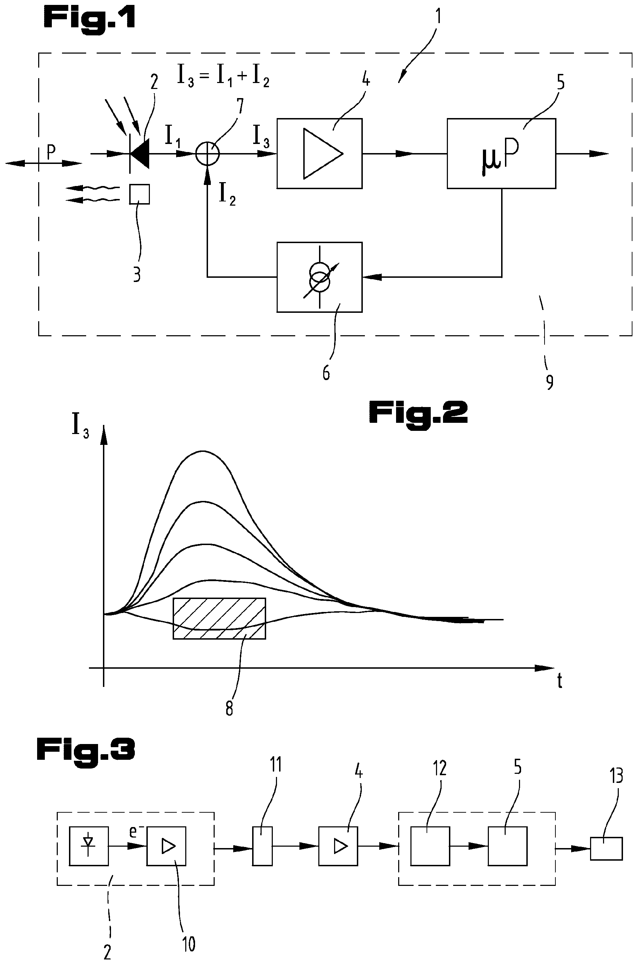

Gemäß der in Fig. 1 dargestellten Ausführungsform der Erfindung kann eine erfindungsgemäße Entfernungsmesseinrichtung einen lichtempfindlichen Empfänger 2 und eine Laserlichtquelle als Sender 3 aufweisen. Sender 3 und Empfänger 2 können ganz oder teilweise in demselben optischen Pfad, der in Fig. 1 durch einen Doppelpfeil und den Buchstaben P angedeutet ist, liegen bzw. verlaufen. D.h. die Sender und Empfänger können koaxial angeordnet sein. Der Empfänger kann als Photodiode, beispielsweise als Lawinendiode ausgeführt sein. Wie aus Fig. 1 weiters ersichtlich ist, kann die Entfernungsmesseinrichtung in bzw. an einem Fernoptischen Gerät 9 angeordnet sein.

Das vom Empfänger/Detektor2 (Lawinen-Photodiode) generierte elektrische Signal (Strom) I1 wird mittels eines Verstärkers 4 verstärkt und von einem Mikroprozessor 5 analysiert. Anstelle eines Mikroprozessors kann auch ein Signalprozessor oder eine andere geeignete programmierbare Schaltung vorgesehen sein. Ferner ist eine gesteuerte Stromquelle 6 zur Erzeugung eines Signals bzw. Stroms I2.

Vor dem Verstärker 4 ist ein Summationsknoten 7 angeordnet, wo die Signale I1 + I2 zu dem Signal I3 überlagert werden, sodass gilt I3=I1+I2.

Der Mikroprozessor 5 bestimmt nach bekannter Art und Weise die zeitliche Lage der reflektierten, von dem Sender 3 ausgesendeten (Licht)Pulse und berechnet daraus die Entfernung 13 zum Ziel. Da die reflektierten Pulse üblicherweise sehr klein und mit viel Hintergrundrauschen behaftet sind, wird zur Verbesserung des Signal-Rausch-Verhältnisses ein ganzer Pulszug (bis zu einigen 1000 Pulsen) durch Mittelung ausgewertet.

Bei jeder Messung werden nun die ersten Pulse dazu verwendet, um den Anteil des optischen Übersprechers (Echosignal) zu bestimmen. Dazu analysiert der Mikroprozessor 5 wie in Fig. 2 dargestellt, ein sehr frühes zeitliches Fenster, in dem ein Teil des optischen Übersprechers (Echosignal) vorhanden ist. Mit dieser Information über die Signalhöhe in diesem Zeitfenster wird über die gesteuerte Stromquelle 6 ein Gegenpuls erzeugt, welcher nun dem Messsignal I1, welches von dem Empfänger 2 erzeugt wird, noch vor dem Verstärker überlagert wird und somit den Anteil des Übersprechers (Echosignal) verkleinert. Der Mikroprozessor 5 wertet dieses neue Signal I3 in diesem Zeitfenster 8 nochmals aus und prüft, ob das Signal I3 sich in diesem zeitlichen Fenster verkleinert hat. Sollte es noch zu groß sein, so wird der Vorgang (bis zu einer maximalen Anzahl von Zyklen, die im Wesentlichen nur durch die zur Verfügung stehende Messzeit begrenzt ist) bei einer weiteren Anpassung der Pulshöhe und der zeitlichen Lage des Gegenpulses I2 wiederholt. D.h. es wird überprüft, ob die Höhe des Signals I3 innerhalb des Zeitfensters 8 einem Sollwert für die Signalhöhe des Signals I3 entspricht, wie sie einem durch keine Reflexionen, Streulicht etc. des Senders gestörten Zustand entspricht. Das Signal I3 wird somit durch Veränderung des Signals I2 auf den Sollwert hin geregelt. Auf diese Weise lässt sich das Echosignal vor der Signalauswertung in dem Mikroprozessor 5 unterdrücken bzw. kompensieren.

Wie in Fig. 3 dargestellt, kann der Empfänger 2 neben der (Lawinen-)Photodiode einen internen Verstärker 10 aufweisen. Das Signal I1 und/oder das Gegensignal I2 und/oder das Signal I3 können in einer Stufe 11 einer Pulsweitenmodulation (PFN) und/oder einer Impedanzanpassung und/oder einer Filterung unterworfen werden, bevor sie dem Verstärker 4 zugeführt werden. Weiters kann dem Mikroprozessor 5 ein Analog/Digital-Wandler 12 vorgeschaltet sein.

Fig. 2: zeigt den Strom I3 nach der Summation. Das Fenster 8, in welchem das Summensignal I3 liegen muss, ist schraffiert dargestellt. Die größte Kurve in Fig. 2 ist ohne Kompensation, dann folgen immer kleiner werdend die Kalibrationsschritte, bis die niedrigste Kurve dann im Fenster liegt => Kalibration abgeschlossen.

Ist dieser Kalibiervorhang abgeschlossen, so erfolgt die eigentliche Messung. Dies hat somit den Vorteil, dass nach Kompensation des Übersprechers Messungen auch bei kurzen Laufzeiten (kurzen Entfernungen) möglich sind und da wenn eine Kompensation vor jeder Messung kalibriert wird, sich zeitlich ändernde Übersprecher (zB. durch Fingerabdrücke auf der Frontlinse, sich absetzende Staubpartikel, sich leicht ändernde optische Pfade durch mechanische oder thermische Einflüsse, sich ändernden Pulsamplitude des Senders) auch berücksichtigt werden können. Hierbei wird angenommen, dass die Pulsform des Übersprechers gleich bleibt. Allgemein ist es aber auch denkbar, die Pulsform in dem Regelkreis variabel zu gestalten und als weiteren Parameter (neben der Pulshöhe und der zeitlichen Lage) in den Regelkreis zur Kompensation mit aufzunehmen.

Alternativ zu oder zusätzlich zu der oben erwähnten Regelung könnte, bei Verwendung einer Lawinen-Photodiode, deren Vorspannung so geändert werden, dass sie während des Starts der Entfernungsmessung nicht "scharf" ist und im Wesentlichen keine Messsignale abgibt. Einen vorgebbaren Zeitraum nach Beginn der Messung könnte dann die Vorspannung so erhöht werden, dass die Lawinen-Photodiode empfindlich genug für die Detektion des von dem Ziel reflektierten Lichtpulses ist.

Auch wäre es möglich, alternativ oder zusätzlich zu den in dem letzten Absatz erwähnten Ausführungsbeispielen, den Verstärkungsfaktor zumindest einer der Stufen des Verstärkers 4 zeitabhängig zu variieren, so könnte bei Beginn der Messung keine Signalverstärkung erfolgen und einen definierten Zeitraum nach Start der Messung die Verstärkung entsprechend erhöht werden.

### Bezugszeichenaufstellung

- 1: Entfernungsmesseinrichtung
- 2: Empfänger
- 3: Sender
- 4: Verstärker
- 5: Mikroprozessor

- 6: Stromquelle
- 7: Summationsknoten
- 8: Zeitfenster
- 9: Fernoptische Vorrichtung
- 10: Interner Verstärker

- 11: Pulsweitenmodulator
- 12: Analog/digital Konverter
- 13: Entfernung zum Ziel

## Patentansprüche

1. Verfahren zum Unterdrücken eines optischen Übersprechers in einer Entfernungsmesseinrichtung (1), welche zumindest einen lichtempfindlichen Empfänger (2) und zumindest eine Laserlichtquelle als Sender (3) aufweist, wobei ein von dem Empfänger (2) generiertes elektrische Signal (I1) mittels eines Verstärkers (4) verstärkt und von einem Mikroprozessor (5) analysiert wird, wobei der Mikroprozessor (5) die zeitliche Lage der reflektierten, von dem Sender (3) ausgesendeten Licht-Pulse bestimmt und daraus eine Entfernung (13) zu einem Ziel berechnet, **dadurch gekennzeichnet, dass** zumindest ein dem optischen Übersprecher entsprechendes Gegensignal (I2) erzeugt und einem von dem Empfänger (2) erzeugten Signal (I1) überlagert und der Anteil des Übersprechers gezielt verkleinert wird, wobei bei jeder Messung erste Pulse dazu verwendet werden, den Anteil des optischen Übersprechers zu bestimmen, wobei der Mikroprozessor (5), ein sehr frühes zeitliches Fenster analysiert, in dem ein Teil des optischen Übersprechers vorhanden ist, mit einer Information über eine Signalhöhe in diesem Zeitfenster (8) wird über eine gesteuerte Stromquelle (6) ein Gegenpuls (I2) erzeugt, welcher dem von dem Empfänger (2) erzeugten Signal (I1) vor dem Verstärker (3) überlagert wird und den Anteil des Übersprechers verkleinert, wobei das neue Signal (I3) durch Veränderung des Gegenpulses (I2) auf einen Sollwert hin geregelt wird.

2. Verfahren nach Anspruch 1, **dadurch gekennzeichnet, dass** das von dem Empfänger (2) erzeugte Signal (I1) und das Gegensignal (I2) additiv oder subtraktiv überlagert werden.

3. Entfernungsmesseinrichtung (1) mit zumindest einem lichtempfindlichen Empfänger (2) und zumindest einer Laserlichtquelle (3) als Sender, wobei die Entfernungsmesseinrichtung dazu eingerichtet ist, ein von dem Empfänger (2) generiertes elektrisches Signal (I1) mittels eines Verstärkers (4) zu verstärken und mittels eines Mikroprozessors (5) zu analysieren, wobei der Mikroprozessor (5) dazu eingerichtet ist, die zeitliche Lage der reflektierten, von dem Sender (3) ausgesendeten Licht-Pulse zu bestimmen und daraus eine Entfernung (13) zu einem Ziel zu berechnen, **dadurch gekennzeichnet, dass** die Entfernungsmesseinrichtung (1) dazu eingerichtet ist, zumindest ein einem optischen Übersprecher entsprechendes Gegensignal (I2) zu erzeugen und einem von dem Empfänger (2) erzeugten Signal (I1) zu überlagern und den Anteil des Übersprechers gezielt zu verkleinern, wobei der Mikroprozessor dazu eingerichtet ist, bei jeder Messung erste Pulse dazu zu verwenden, den Anteil des optischen Übersprechers zu bestimmen und ein sehr frühes zeitliches Fenster zu analysieren, in dem ein Teil des optischen Übersprechers vorhanden ist, wobei die Entfernungsmesseinrichtung dazu eingerichtet ist, mit einer Information über eine Signalhöhe in diesem Zeitfenster (8) über eine gesteuerte Stromquelle (6) einen Gegenpuls (I2) zu erzeugen und diesen Gegenpuls (I2) dem von dem Empfänger (2) erzeugten Signal (I1) vor dem Verstärker (3) zu überlagern und den Anteil des Übersprechers zu verkleinern und das neue Signal (I3) durch Veränderung des Gegenpulses (I2) auf einen Sollwert hin zu regeln.

4. Entfernungsmesseinrichtung, nach Anspruch 3, **dadurch gekennzeichnet, dass** sie dazu eingerichtet ist, das von dem Empfänger (2) erzeugte Signal (I1) und das Gegensignal (I2) additiv oder subtraktiv zu überlagern.

5. Fernoptische Vorrichtung (9), insbesondere Fernrohr oder Zielfernrohr, **dadurch gekennzeichnet, dass** sie eine Entfernungsmesseinrichtung (1) nach einem der Ansprüche 3 oder 4 aufweist, wobei Sender (2) und Empfänger (3) zumindest teilweise in demselben optischen Pfad (P) angeordnet sind.

## Claims

1. A method for suppressing an optical crosstalk in a rangefinder device (1), which has at least one light-sensitive receiver (2) and at least one laser light source as a transmitter (3), wherein an electrical signal (I1) generated by the receiver (2) is amplified by means of an amplifier (4) and analyzed by a microprocessor (5), wherein the microprocessor (5) determines the temporal position of the reflected light pulses sent by the transmitter (3) and therefrom calculates a distance (13) to a target, **characterized in that** at least one counter signal (12) corresponding to the optical crosstalk is generated and is superimposed to a signal (I1) generated by the receiver (2) and the portion of the crosstalk is specifically reduced , wherein in each measurement, first pulses are used for determining the portion of the optical crosstalk, wherein the microprocessor (5) analyzes a very early temporal window in which a part of the optical crosstalk is present, a counter pulse (12) is generated with an information on a signal level in this time window (8) via a controlled power source (6), said counter pulse (12) being superimposed to the signal (I1) generated by the receiver (2) upstream of the amplifier (3) and reducing the portion of the crosstalk, wherein the new signal (13) is controlled to correspond to a target value by amendment of the counter pulse (12).

2. The method according to claim 1, **characterized in that** the signal (I1) generated by the receiver (2) and the counter signal (12) are superimposed in an additive or subtractive manner.

3. A rangefinder device (1) having at least one light-sensitive receiver (2) and at least one laser light source (3) as a transmitter, wherein the rangefinder device is designed for amplifying an electrical signal (I1) generated by the receiver (2) by means of an amplifier (4) and analyzing it by means of a microprocessor (5), wherein the microprocessor (5) is designed for determining the temporal position of the reflected light pulses sent by the transmitter (3) and therefrom calculating a distance (13) to a target, **characterized in that** the rangefinder device (1) is designed for generating at least counter signal (12) corresponding to an optical crosstalk and superimposing it to the signal (I1) generated by the receiver (2) and specifically reducing the portion of the crosstalk, wherein the microprocessor is designed for using first pulses, in each measurement, to determine the portion of the optical crosstalk and for analyzing a very early temporal window, in which a part of the optical crosstalk is present, wherein the rangefinder device is designed for generating a counter pule (12) with an information on a signal level in this time window (8) via a controlled power source (6) and for superimposing said counter pulse (12) to the signal (I1) generated by the receiver (2) upstream of the amplifier (3) and for reducing the share of the crosstalk and for controlling the new signal (13) to correspond to a target value by amendment of the counter pulse (12).

4. The rangefinder device according to claim 3, **characterized in that** it is designed for superimposing the signal (I1) generated by the receiver (2) and the counter signal (12) in an additive or subtractive manner.

5. A long-range optical device (9), in particular a telescope or sighting telescope, **characterized in that** it comprises a rangefinder device (1) according to one of claims 3 or 4, wherein the transmitter (2) and the receiver (3) are at least partly arranged in the same optical path (P).

## Revendications

1. Procédé d'élimination d'une diaphonie optique dans un dispositif de mesure de distance (1), qui comprend un récepteur photosensible (2) et au moins une source de lumière laser en tant qu'émetteur (3), un signal électrique (I1) généré par le récepteur (2) étant amplifié au moyen d'un amplificateur (4) et analysé par un microprocesseur (5), le microprocesseur (5) déterminant la position temporelle des impulsions lumineuses émises par l'émetteur (3) et calculant, à partir de celle-ci, une distance (13) par rapport à une cible, **caractérisé en ce qu'**au moins un contre-signal (I2) correspondant à la diaphonie optique est généré et superposé à un signal (I1) généré par le récepteur (2) et 1 part de la diaphonie est réduite de manière ciblée, moyennant quoi, à chaque mesure, des premières impulsions étant utilisées pour déterminer la part de la diaphonie optique, le microprocesseur (5) analysant une fenêtre temporelle très précoce dans laquelle une partie de la diaphonie optique est présente, une contre-impulsion (I2), avec une information concernant un niveau de signal dans cette fenêtre temporelle (8) étant générée par l'intermédiaire d'une source de courant contrôlée (6), qui est superposée, avant l'amplificateur (3), au signal (I1) généré par le récepteur (2) et qui réduit la part de diaphonie, le nouveau signal (I3) étant régulé en fonction d'une valeur de consigne par la modification de la contre-impulsion (I2).

2. Procédé selon la revendication 1, **caractérisé en ce que** le signal (I1) généré par le récepteur (2) et le contre-signal (I2) sont superposés de manière additive ou soustractive.

3. Dispositif de mesure de distance (1) avec au moins un récepteur photosensible (2) et au moins une source de lumière laser (3) en tant qu'émetteur, le dispositif de mesure de distance étant conçu pour amplifier un signal électrique (I1) généré par le récepteur (2) au moyen d'un amplificateur (4) et pour l'analyser au moyen d'un microprocesseur (5), le microprocesseur (5) étant conçu pour déterminer la position temporelle des impulsions lumineuses réfléchies émises par l'émetteur (3) et pour calculer, à partir de celle-ci, une distance (13) par rapport à une cible, **caractérisé en ce que** le dispositif de mesure de distance (1) est conçu pour générer au moins un contre-signal (I2) correspondant à une diaphonie optique et pour le superposer à un signal (I1) généré par le récepteur (2) et pour réduire de manière ciblée la part de diaphonie, le microprocesseur étant conçu pour utiliser, à chaque mesure, des premières impulsions pour déterminer la part de diaphonie optique et pour analyser une fenêtre temporelle très précoce dans laquelle une partie de la diaphonie optique est présente, le dispositif de mesure de distance étant conçu pour générer une contre-impulsion (I2), avec une information concernant un niveau de signal dans cette fenêtre temporelle (8), par l'intermédiaire d'une source de courant contrôlée (6), et pour la superposer, avant l'amplificateur (3), au signal (I1) généré par le récepteur (2) et pour réduire la part de diaphonie et pour réguler le nouveau signal (I3) en fonction d'une valeur de consigne par la modification de la contre-impulsion (I2).

4. Dispositif de mesure de distance selon la revendication 3, **caractérisé en ce qu'**il est conçu pour superposer le signal (I1) généré par le récepteur (2) et le contre-signal (I2) de manière additive ou soustractive.

5. Dispositif de téléoptique (9), plus particulièrement télescope ou lunette de visée, **caractérisé en ce qu'**il comprend un dispositif de mesure de distance (1) selon l'une des revendications 3 ou 4, l'émetteur (2) et le récepteur (3) étant disposés au moins partiellement sur le même trajet optique (P).
